Europäisches Patentamt

European Patent Office

Office européen des brevets

(11) Publication number: **0 451 040 A1**

(12) **EUROPEAN PATENT APPLICATION**

(21) Application number: **91400879.2**

(22) Date of filing: **29.03.91**

(51) Int. Cl.⁵: **A61M 25/00, A61M 39/00**

(30) Priority: **03.04.90 US 504133**

(43) Date of publication of application:
**09.10.91 Bulletin 91/41**

(84) Designated Contracting States:
**AT BE CH DE DK ES FR GB GR IT LI LU NL SE**

(71) Applicant: **Parker, Robert L.
661 Crossing Creek Point
Austell, Georgia 30001 (US)**
Applicant: **Knutson, Richard A.
130 North Shelby Street
Greenville, MS 38701 (US)**

(72) Inventor: **Parker, Robert L.
661 Crossing Creek Point
Austell, Georgia 30001 (US)**
Inventor: **Knutson, Richard A.
130 North Shelby Street
Greenville, MS 38701 (US)**

(74) Representative: **Phélip, Bruno et al
c/o Cabinet Harlé & Phélip 21, rue de La
Rochefoucauld
F-75009 Paris (FR)**

(54) **Closed system intravenous catheter.**

(57) An improved intravenous catheter is disclosed which, when properly positioned within a vein, provides a closed system catheter. The closed system intravenous catheter comprises a catheter having a tapered portion (20), a hub portion (36) and a fluid-flow passageway (28) extending therethrough. A fluid-impermeable elastomeric gasket member (30) is disposed within the hub portion of the catheter for sealing the hub portion and the fluid-flow passageway of the catheter. A skin-penetrating stylet (37) of a stylet assembly (32) extends through the elastomeric gasket member and the fluid-flow passageway of the catheter so hat a puncture tip (38) on a proximal end of the skin-penetrating stylet extends outwardly from the tapered portion of the catheter. An air-permeable, liquid-impermeable vent assembly (80) is connected to an observation chamber (74) of the stylet assembly so that blood flow into a flashpoint cavity (76) defined by the observation chamber can be observed and controlled. The vent also permits auxiliary equipment to be connected without leakage of blood. A retractable sheath (130) for the skin-penetrating stylet is also provided which covers the skin-penetrating stylet when same is removed from the catheter so as to minimize exposure of health care workers to the potentially contaminated puncture tip of the skin-penetrating stylet.

Fig. 3

EP 0 451 040 A1

## 1. Field of the Invention.

The present invention relates generally to catheters used to administer intravenous, arterial or central venous fluids, and more particularly but not by way of limitation, to an improved closed system angiocatheter.

## 2. Discussion of Prior Art.

Catheters are used to administer intravenous, arterial or central venous fluids to a patient. One of the most commonly employed catheters is the angiocatheter which comprises a tapered catheter having a fluid-flow passageway extending therethrough and a skin-penetrating stylet. The skin-penetrating stylet is provided with a sharpened puncture tip and an observation chamber having a flashpoint cavity therein. When the skin-penetrating stylet is positioned within the fluid-flow passageway of the catheter, the puncture tip extends beyond the catheter so that the puncture tip can puncture the skin and vein prior to placement of the catheter into the vein. Thus, blood can flow through the skin-penetrating stylet and into the flashpoint cavity to indicate to the health care worker that the angiocatheter is properly positioned within the vein. Further confirmation that the angiocatheter has been properly positioned in the vein can be achieved by partially "backing off" or removing the skin-penetrating stylet so that blood flow can continue through the fluid- flow passageway of the catheter.

When blood flow indicates proper positioning of the catheter, and the skin-penetrating stylet is removed therefrom, intravenous tubing can be connected to the catheter so that intravenous fluids can be administered to the patient. To prevent backflow of blood, the health care worker places a thumb against the vein to tamponade the vein and thereby restrict blood flow into the catheter.

Because it is desirable to maintain the catheter in place, even when not administering an intravenous fluid, externally disposed cap members have heretofore been employed to seal the catheter in order to prevent infectious germs and viruses from entering the patient's bloodstream through the catheter. As can be appreciated, germs and viruses often enter the bloodstream because of the manipulation required in attempting to remove and reseal the catheter which results in potential infection. Further, blood often leaks through the catheter and into contact with the attending health care worker so that the health care worker is exposed to potentially infectious blood. In addition, the blood gets onto the bedding of the patient prior to capping of the catheter. This blood leakage can be dangerous, especially if the blood is infectious, resulting in potential transfer of the infection. In addition, blood leakage through the catheter results in unnecessary work, such as changing the bedding of the patient. Thus, leakage of blood through the catheter is very undesirable and potentially dangerous.

Prior attempts to provide a "closed system" catheter which would prevent germs and viruses from entering the patient's bloodstream, as well as prevent leakage of blood through the catheter after removal of the skin-penetrating stylet or prior to the attachment of intravenous tubes and the like, have not been successful. Thus, the need remains for the development of a closed system catheter which would enable one to withdraw the skin-penetrating stylet from the catheter without leakage of blood through the implanted catheter, and which would permit one to connect and disconnect intravenous tubing to the catheter without exposing the health care worker, as well as auxiliary personnel such as laundry workers and the like, to potentially infectious blood; while at the same time preventing exposure of the internal portion of the catheter, and thus the patient, to potentially infectious germs or viruses present in the surrounding environment. Further, in order to reduce the number of punctures which a patient is subjected to, it would be highly desirable if one could provide an improved angiocatheter which prevents back flow of blood and yet readily permits the health care worker to obtain blood samples through the catheter without leakage of blood or the necessity of capping or removing a cap from the catheter. It is to such an improved closed system catheter that the present invention is directed.

## Summary of the Invention

According to the present invention, an improved intravenous catheter is provided which, when properly positioned within a vein, provides a closed system catheter. That is, once the catheter is implanied in a vein, the catheter is effectively self- sealing and the catheter not only prevents leakage of blood therethrough, but also effectively closes off the internal portion of the catheter to the external surrounding environment so that germs and viruses cannot enter the patient's bloodstream through the implanted catheter.

Broadly, the closed system intravenous catheter of the present invention comprises a catheter having a tapered portion, a hub portion and a fluid-flow passageway extending therethrough. A fluid-impermeable elastomeric gasket member is disposed within the hub portion of the catheter for sealing the hub portion and the fluid-flow passageway of the catheter. A skin-penetrating stylet of a stylet assembly extends through the elastomeric gasket member and the fluid-flow passageway of the catheter so that a puncture tip on a proximal end of the skin-penetrating stylet extends outwardly from the tapered portion of the catheter. The puncture tip of the skin-penetrating stylet permits one to produce a puncture site in the patient's skin so that the tapered portion of the catheter can be positioned in a vein without great discomfort to the patient.

To insure that the catheter has been properly placed in the vein, the stylet assembly is provided with an observation chamber connected to a distal end of the skin-penetrating stylet. The observation chamber, which defines a flashpoint cavity, is connected to an air-permeable, liquid-impermeable vent assembly so that the flow of blood into the flashpoint cavity can be observed and effectively controlled. The vent assembly not only permits one to control the flow of blood into the flashpoint cavity, but also permits one to operably connect auxiliary equipment, such as a blood sampling device, to the catheter without leakage of blood.

In order to accomplish the before-mentioned dual function the vent assembly comprises an air-permeable, liquid-impermeable frit supported on the observation chamber of the stylet assembly and a vent stylet connected to the observation chamber of the stylet assembly so as to openly communicate with the flashpoint cavity. A fluid-impermeable elastomeric cover member is connected to the frit such that the cover member is positionable over the vent stylet. The air-permeable, liquid-impermeable frit cooperates with the vent stylet and the elastomeric cover member so that blood is permitted to flow into the flashpoint cavity of the observation chamber, while preventing the flow of blood from the flashpoint cavity through the vent stylet until such time as auxiliary equipment has been properly connected to the observation chamber of the stylet assembly.

To connect auxiliary equipment, such as a blood sampling device to the angiocatheter of the present invention, the fluid-impermeable elastomeric cover member is compressed by the auxiliary equipment so that a puncture tip of the vent stylet penetrates through the elastomeric cover member and fluid communication is established between the stylet assembly and the auxiliary equipment via the vent stylet. Because of the resilient nature of the fluid-impermeable elastomeric cover member, when the auxiliary equipment is disengaged from the vent stylet, the cover member returns to a non-compressed condition and effectively seals the vent stylet so that the fluid-tight seal is reestablished.

To prevent exposure of a health care worker to inadvertent puncture by the vent stylet during placement of the angiocatheter into the vein of a patient, as well as to protect the vent stylet, a closure member or cap is positionable over the vent assembly so as to effectively encapsulate the vent stylet.

To prevent exposure of a health care worker from inadvertent puncture by the skin-penetrating stylet used to puncture the skin and vein for vein entry of the catheter after the skin-penetrating stylet has been removed from the catheter, a protective sheath is advantageously incorporated into the stylet assembly of the angiocatheter so as to effectively encapsulate the puncture tip of the skin-penetrating stylet.

An object of the present invention is to provide an improved intravenous catheter.

Another object of the present invention, while achieving the before stated object, is to provide a closed system intravenous catheter which prevents undesired leakage of blood therethrough and which prevents exposure of health care workers to potentially infectious blood.

Another object of the present invention, while achieving the before stated objects, is to provide a multi-purpose closed system intravenous catheter which is easy to use and which reduces the number of puncture sites required in the treatment of a patient.

Yet another object of the present invention, while achieving the before stated objects, is to provide an improved closed system catheter which is simple in construction and economical to manufacture.

Other objects, advantages and features of the present invention will become apparent from the following detailed description when read in conjunction with the appended claims.

Brief Description of the Drawings

FIG. 1 is a pictorial illustration of an angiocatheter of the present invention disposed within a vein of a patient's arm.

FIG. 2 is an enlarged side view, partially in cross section, of the angiocatheter of the present invention having a closure cap supported on an observation chamber so as to be disposed in a covering position over a vent assembly of the angiocatheter.

FIG. 3 is an enlarged side view, partially in cross section, of the catheter of the angiocatheter of the present invention illustrating auxiliary equipment positioned for attachment to the catheter.

FIG. 4 is an enlarged view of the catheter of the angiocatheter of the present invention, taken along the line 4-4 of FIG. 3.

FIG. 5 is an enlarged side view, partially in cross section, of the angiocatheter of the present invention illustrating a vent assembly for the observation chamber of the stylet assembly wherein the closure cap is disposed in a covering position over the vent assembly.

FIG. 6 is an enlarged, fragmental side view, partially in cross section, of the vent assembly of the angiocatheter of the present invention wherein an elastomeric stylet covering member of the vent assembly is disposed over a vent stylet.

FIG. 7 is an enlarged, fragmental side view, partially in cross section, of a blood sampling device operably connected to the angiocatheter of the present invention.

FIG. 8 is an enlarged, fragmental side view, partially in cross section of a protective sheath for the skin-penetrating stylet of the angiocatheter of the present invention wherein the protective sheath is in a retracted, compressed position when the skin-penetrating stylet is disposed within the catheter.

FIG. 9 is an enlarged, fragmental side view, partially in cross section of the protective sheath for the skin-penetrating stylet of the angiocatheter of the present invention wherein the protective sheath is in an extended skin-penetrating stylet covering position.

FIG. 10 is an enlarged, fragmental side view, partially in cross section of a second embodiment of a protective sheath of the skin-penetrating stylet of the angiocatheter of the present invention.

Detailed Description

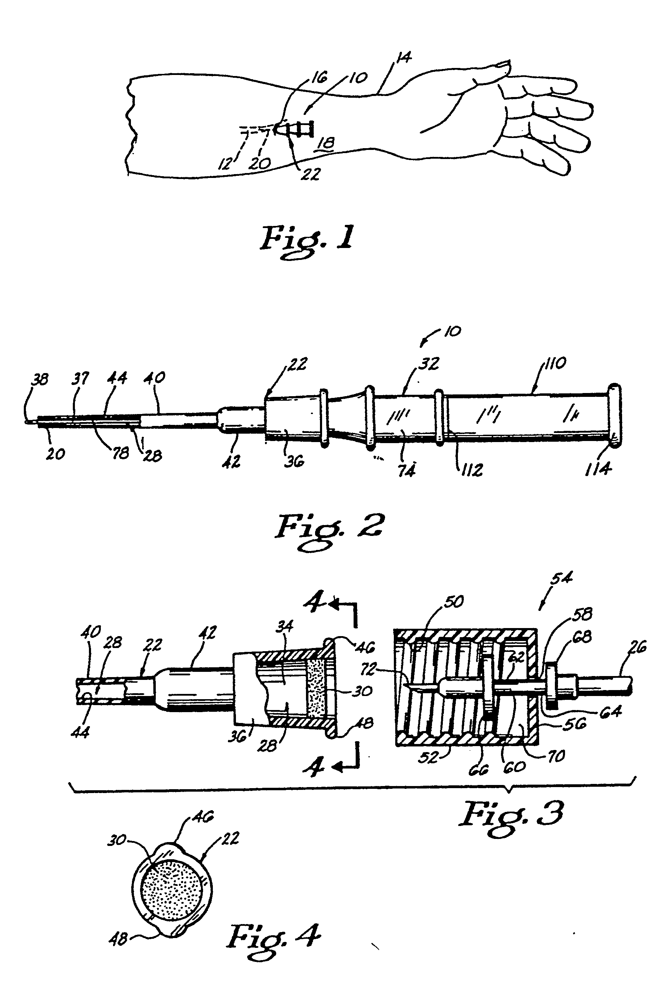

Referring to the drawings, and more particularly to FIG. 1, an improved angiocatheter 10 of the present invention is illustrated positioned within a vein 12 in a patient's arm 14. As will be more fully described hereinafter, the angiocatheter 10 permits one to produce a puncture site 16 in a patient's skin 18 and the underlying vein 12 so that a tapered proximal end portion 20 of a catheter 22 can be positioned within the vein 12 without great discomfort to the patient, while at the same time reducing slippage and coring.

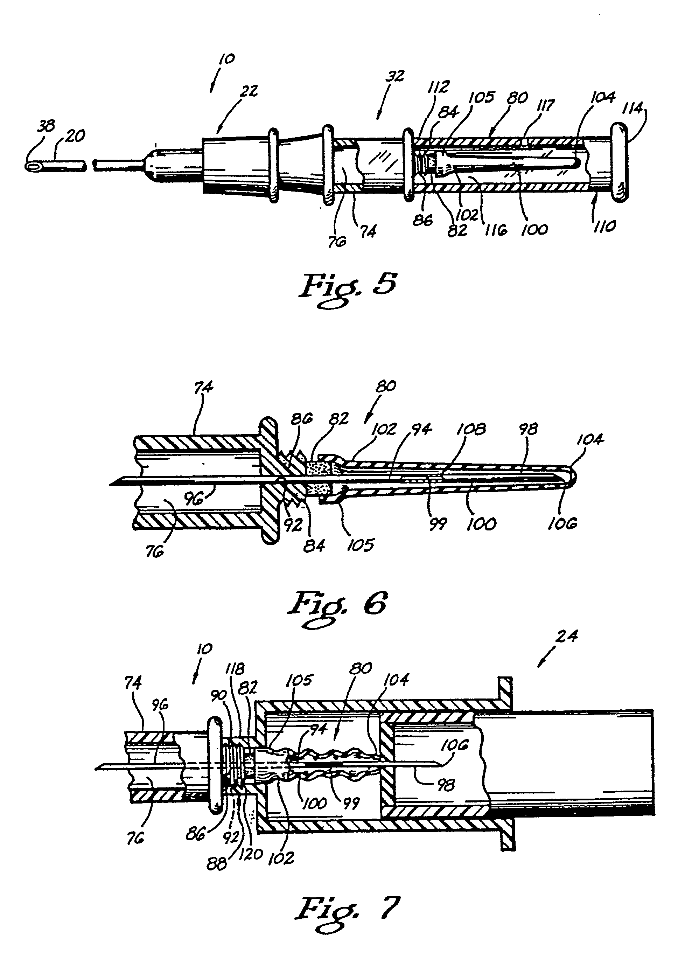

Auxiliary equipment, such as a Vacutainer® brand blood sampling device 24 (FIG. 7), can be connected to the angiocatheter 10 without the need of puncturing the patient's skin a second time to produce a second puncture site, or a standard intravenous needle by be used to connect intravenous fluid tubing 26 (FIG. 3) to the catheter 22 so that intravenous fluids can be administered to the patient via the catheter 22. Further, the unique design of the catheter 22 also permits one to administer repeated injections using an injection needle connected to a syringe.

The term "closed system catheter" as used herein is understood to mean that once the catheter 22 is properly implanted in the vein 12 a fluid-flow passageway 28 which extends through the catheter 22 (FIG. 3) is effectively self-sealing so as to prevent leakage of blood therethrough without the requirement of an external cap member. Thus, the fluid-flow passageway 28 of the catheter 22 is effectively sealed to the surrounding environment so that air, germs and viruses cannot enter the patient's blood-stream through the fluid-flow passageway 28 of the implanted catheter 22.

Referring now to FIGS. 2-5, the angiocatheter 10 comprises the catheter 22, a resilient, fluid-impermeable gasket member 30 and a stylet assembly 32. The resilient gasket member 30, which is fabricated of a substantially fluid-impermeable elastomeric material, is supported with a cavity 34 defined by a hub 36 of the catheter 22 so that the gasket member 30 effectively seals the fluid-flow passageway 28 of the catheter 22 to both air and liquid.

A skin-penetrating stylet 37 of the stylet assembly 32 is slidably disposed within the fluid-flow passageway 28 of the catheter 22 so as to penetrate the resilient gasket member 30. A puncture tip 38 of the skin-penetrating stylet 37 extends outwardly from the tapered proximal end portion 20 of the catheter 22 substantially as shown in FIG. 2. Thus, when positioning the catheter 22 into the vein 12 the puncture tip 38 of the skin-penetrating stylet 37 punctures the puncture site 16 on the patient's skin 18, and the underlying vein 12, so that the tapered proximal end portion 20 of the catheter 22 can be positioned within the vein 12.

When the catheter 22 has been positioned within the vein 12 and the stylet assembly 32 removed therefrom, the resilient gasket member 30 effectively reseals the place of penetration of the skin-penetrating stylet 37 in the resilient gasket member 30 so as to provide a fluid-impermeable seal for the fluid-flow passageway 28 of the catheter 22. Thus, the resilient gasket member 30 prevents blood leakage through the fluid-flow passageway 28 of the catheter 22, while at the same time closing off the fluid-flow passageway 28 from contact with the outward surrounding environment or atmosphere.

As more clearly shown in FIGS. 2 and 3, the catheter 22 comprises the hub 36 and a tapered body member 40. The hub 36 and the tapered body member 40 can be fabricated of any suitable polymeric material; and the hub 36 and the tapered body member are desirably of unitary construction.

The tapered body member 40 is an elongated member having the tapered proximal end portion 20, a distal end portion 42 and a fluid-flow bore 44 extending therethrough. The hub 36 is supported by the distal end portion 42 of the tapered body member 40 such that the cavity 34 of the hub 36 openly communicates with the fluid-flow bore 44 of the tapered body member 40. Thus, the cavity 34 of the hub 36 and the fluid-flow bore 44 of the tapered body member 40 cooperate to define the fluid-flow passageway 28 of the catheter 22.

To assist in the connection of auxiliary equipment such as the intravenous tubing 26 to the catheter 22 so that intravenous fluid can be administered to the patient via the catheter 22 when the stylet assembly 32 has been removed from the catheter 22, the hub 36 of the catheter 22 is further provided with a pair of oppositely disposed, outwardly extending tab members 46, 48 adapted to operably engage mating threads 50 disposed within an open first end 52 of an intravenous fluid tubing connector 54 (see FIG. 3).

The connector 54, a substantially cylindrical-shaped member, in addition to the open first end 52, is characterized as having an opposed second end 56. The opposed second end 56 is provided with a centrally disposed aperture 58 adapted to receive an elongated, cylindrical-shaped tubular coupling member 60.

The coupling member 60 (which is slidably disposed through the aperture 58) is provided with a first end 62 and an opposed second end 64. The first end 62 of the coupling member 60 extends inwardly into the connector 54; and the opposed second end 64 of the coupling member 60 is positioned exterior the connector 54 substantially as shown in FIG. 3. To restrict the to and fro movement of the tubular coupling member 60 through the aperture 58, the coupling member 60 is provided with a pair of spatially disposed stop members 66 and 68, each of which has a diameter greater than the diameter of the aperture 58. As shown in FIG. 3, the stop member 66 is connected to the coupling member 60 so as to be disposed within a hollow interior portion 70 of the connector 54; and the stop member 68 is connected to the coupling member 60 so as to be disposed exterior the connector 54. Thus, when the stop member 68 abuttingly engages the opposed second end 56 of the connector 54, the movement of the coupling member 60 in the "to" direction is halted; whereas, when the stop member 66 abuttingly engages the opposed second end 56 of the connector 54, the movement of the coupling member 60 in the "fro" direction is halted.

In order to establish fluid communication between the intravenous fluid tubing 26 and the fluid-flow passageway 28 of the catheter 22, an intravenous tubing stylet or needle 72 is connected to the first end 62 of the coupling member 60; and the intravenous fluid tubing 26 is connected to the opposed second end 64 of the coupling member 60. Thus, fluid communication is established between the intravenous tubing stylet or needle 72 and the intravenous fluid tubing 26. As can be appreciated, in order to establish fluid communication between the intravenous tubing stylet 72 and the fluid-flow passageway 28 of the catheter 22, the intravenous tubing stylet 72 must have a sufficient length so that when the connector 54 is threadably connected to the hub 36 of the catheter 22, the intravenous tubing stylet 72 penetrates the resilient gasket member 30 and openly communicates with the fluid-flow passageway 28 of the catheter 22.

As shown in FIGS. 2 and 5, the stylet assembly 32, in addition to the skin-penetrating stylet 37, comprises an observation chamber 74 which defines a flashpoint cavity 76 therein. The flashpoint cavity 76 is in fluid communication with a fluid-flow bore 78 of the skin-penetrating stylet 37 so that blood flow through the skin-penetrating stylet 37 via the bore 78 can be observed in the flashpoint cavity 76. A vent assembly 80 is connected to and supported by the observation chamber 74 so that the flow of blood into the flashpoint cavity 76 cannot only be observed therein, but can be effectively controlled by the vent assembly 80. Further, as will be set forth in more detail hereinafter, the vent assembly 80, in addition to permitting one to control the flow of blood into the flashpoint cavity 76, also permits one to operably connect auxiliary equipment, such as the blood sampling device 24 (see Fig. 7) to the stylete assembly 32, and thus the catheter 22 without leakage of blood therethrough.

Referring more specifically to FIGS. 5-7, the vent assembly 80 and its interconnection with the flashpoint cavity 76 of the observation chamber 74 is illustrated. In order to accomplish the before-mentioned dual function, the vent assembly 80 comprises an air-permeable, liquid-impermeable frit member 82 supported on a distal end 84 of the observation chamber 74. The distal end portion 84 of the observation chamber 74 comprises an externally threaded post member 86 adapted to matingly engage a female member 88 having internally disposed threads 90 of an auxiliary piece of equipment, such as the blood sampling device 24. The threaded post member 86 is provided with a centrally disposed bore 92 extending therethrough, the bore 92 adapted to receive a vent stylet 94 such that one end 96 of the vent stylet 94 extends inwardly into the observation chamber 74 and an opposed second or distal end 98 thereof extends outwardly from the threaded post member 86 of the observation chamber 74. Thus, the vent stylet 94 openly communicates with the flashpoint cavity 76 defined by the observation chamber 74 via a fluid-flow passage bore 99 of the vent stylet 94.

The vent assembly 80 further comprises a fluid-impermeable elastomeric cover member 100 supported by the frit member 82 such that a fluid-tight seal is formed therebetween. The cover member 100 is a tubular member and is positionable over the vent stylet 94 substantially shown. Thus, the cover member 100 is characterzed as having an open first end portion 102 and a closed second end portion 104. In order to secure the cover member 100 to the frit member 82, the first end portion 102 is provided with a skirt 105 adapted to frictionally engage a portion of the frit member 82 such that the fluid-tight seal is formed therebetween. In connecting the elastomeric cover member 100 to the frit member 82, care should be exercised to insure that sufficient surface area of the frit member 82 remains uncovered by the skirt 105 so that air can be expelled from the observation chamber 74 via the vent stylet 94 and the frit member 82 when blood is permitted to flow into the flashpoint cavity 76 of the observation chamber 74. Thus, the frit member 82 cooperates with the vent stylet 94 and the elastomeric cover member 100 so that blood is permitted to flow into the flashpoint cavity 76 of the observation chamber 74 via the skin-penetrating stylet 37 when the skin-penetrating stylet 37 and the catheter 22 are properly positioned within a vein; while preventing the flow of blood from the flashpoint cavity 76 through the vent stylet 94 until such time as auxiliary equipment, such as the blood sampling device 24, has been properly connected to the threaded post member 86 of the stylet assembly 32.

It should be noted that the cover member 100 is provided with a length such that when the cover member 100 is positioned over the vent stylet 94, the closed second end 104 thereof is disposed substantially adjacent a puncture tip 106 of the vent stylet 94 but in a non-blocking relationship therewith so that fluid flow can be maintained through the fluid-flow passageway 99 of the vent stylet 94. Thus, as blood enters the flashpoint cavity 76 of the observation chamber 74, air is expelled from the flashpoint cavity 76 via the fluid-flow passageway 99 of the vent stylet 94. The air expelled from the vent stylet 94 travels along a passageway 108 formed between the vent stylet 94 and the cover member 100 so as to be directed to the frit member 82 where the air, upon passage therethrough, is discharged to the surrounding environment.

While the cover member 100 has been depicted as being connectable to the frit member 82 via the skirt 105 formed on the first end portion 102 of the cover member 100, any suitable means for connecting the first end portion 102 of the cove member 100 to the frit member 82 so as to form a fluid-tight seal therebetween can be employed. For example, one can employ a suitable adhesive, exercising care to insure that the frit maintains sufficient porosity for the passage of air therethrough, an O-ring, a clamp, or other similar connecting devices.

To prevent exposure of a health care worker to inadvertent puncture by the puncture tip 106 of the vent stylet 94 during placement of the angiocatheter 10 into the vein of a patient, as well as to protect the vent stylet 94 from unnecessary exposure to the environment and potential damage, the vent assembly 80 further comprises a closure member 110 positionable over the vent stylet 94 substantially as shown in FIGS. 1, 2 and 5. The closure member 110 is a cylindrical-shaped member having a first end 112, a closed second end 114 and a cavity 116 openly communicating with the first end 112. To enhance discharge of air from the flashpoint cavity 76 via the vent stylet 94 and the frit member 82, the closure member 110 is desirably provided with at least one air port 117 substantially as shown in FIG. 5. Thus, the closure member 110 can be disposed in a covering position over the vent stylet 94, the elastomeric cover member 100 and the frit member 82 so that the first end 112 supportingly engages the observation chamber 74. Desirably, the closure member 110 frictionally engages the threaded post member 86 so that the cover member 110 is secured in a covering position, while permitting the closure member 110 to be readily removed when access is desired to the vent stylet 94.

When it is determined desirable to attach auxiliary equipment to the angiocatheter 10, such as the blood sampling device 24 (FIG. 7), the closure member 110 is removed so that the vent stylet 94 and the cover member 100 are accessible. The auxiliary equipment, such as the blood sampling device 24, is provided with a throat portion 118 having internally disposed threads 120. When the throat portion 118 is positioned over the vent stylet 94, and the auxiliary equipment is threadably connected to the threaded post member 86 of the observation chamber 74, the throat portion 118 of the device 24 is disposed in a covering position over the frit member 82. Further, when the blood sampling device 24 is threadedly connected to the threaded post member 86, the vent stylet 94 is forced to penetrate the closed second end 104 of the covering member 100 and thereby establish fluid communication with the auxiliary equipment. That is, the blood sampling device 24 compresses the cover member 100 so as to permit penetration of the puncture tip 106 of the vent stylet 94 therethrough, and thereby allows blood to flow from the catheter to the blood sampling device 24.

Because of the elastic properties of the cover member 100, when the blood sampling device 24 is removed from the angiocatheter 10, the cover member 100 returns to its non-compressed condition and once again serves to restrict liquid flow through the vent stylet 94. Further, the elastomeric characteristics of the cover member 100 permit the point of puncture through the closed second 104 thereof to reseal itself so that an effective fluid-impermeable seal is again achieved.

The frit member 82 of the vent assembly 80 is, as previously stated, air-permeable and liquid-impermeable. Thus, the frit member 82 can be fabricated of any suitable material having those 'required properties. For example, the frit member 82 can be fabricated of a ceramic material, non-wicking fibrous material, compressed fibrous polymeric material and the like.

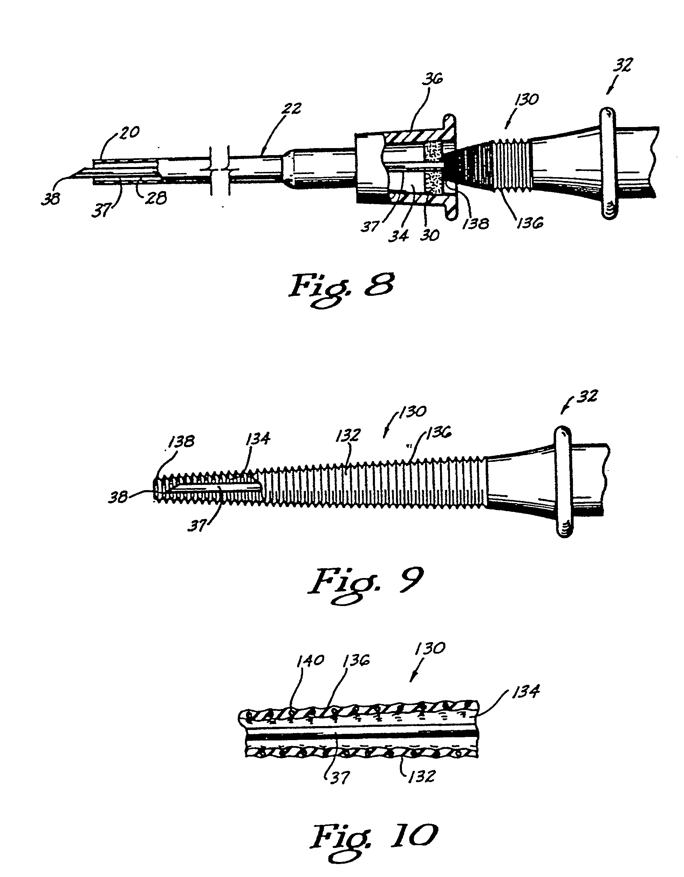

Referring now to FIGS. 8 and 9, the angiocatheter 10 further comprises a retractable sheath 130 connected to and supported by the observation chamber 74 of the stylet assembly 32 so as to minimize exposure of a health care worker to inadvertent puncture by the puncture tip 38 of the skin-penetrating stylet 37 when the skin-penetrating stylet 37 is removed from the passageway 28 of the catheter 22. The retractable sheath 130 comprises an elongated body member 132 having a longitudinally extending passageway 134 adapted to receive the skin-penetrating stylet 37 of the stylet assembly 32; and the retractable sheath 130 is selectively movable between a retracted position (FIG. 8) and an extended position (FIG. 9).

The body member 132 is preferably fabricated of a polymeric material having sufficient rigidity to maintain the body member 132 of the retractable sheath 130 in a substantially rigid, extended position, while at the same time permitting the body member 132 of the retractable sheath 130 to be slidably moved to the retracted position along the skin-penetrating stylet 37 when the skin-penetrating stylet 37 of the stylet assembly 32 is slidably positioned in the fluid-flow passageway 28 of the catheter 22. Further, the polymeric material desirably possesses sufficient memory propercies so that the sheath 130 returns to its extended position when compressive forces maintaining the sheath 130 in its retracted position are removed.

The body member 132 of the retractable sheath 130 is provided with a length greater than the length of the skin-penetrating stylet 37 when same is in the extended position (see FIG. 9) so that the puncture tip 38 of the skin-penetrating stylet 37, as well as the skin-penetrating stylet 37, are completely surrounded by the body member 132 of the retractable sheath 130.

Further to enhance the selective movement of the retractable sheath 130 between the extended position and the retracted position, the body member 132 of the retractable sheath can be provided with a plurality of accordian-like pleats 136 formed along its length. Thus, when the skin-penetrating stylet 37 is positioned within the fluid-flow passageway 28 of the catheter 22, the gasket member 30 exerts a force on an adjacently disposed end 138 of the body member 132 and continued movement of the skin- penetrating stylet 37 through the passageway 28 of the catheter 22 results in compression and folding of the accordian-like pleats 136 substantially as shown in FIG. 8.

When it is determined desirable to remove the stylet assembly 32 from the catheter 22, the reduced force applied to the stylet assembly 32 during withdrawal relaxes or releases the compressive tension on the adjacently disposed end 138 of the body member 130 caused by the gasket member 30 so that the body member 132 of the sheath 130 is allowed to return to its extended position as the skin-penetrating stylet 37 is withdrawn from the fluid-flow passageway 28 of the catheter 22. When the skin-penetrating stylet 37 has been completely withdrawn from the fluid-flow passageway 28 of the catheter 22, the body member 132 of the sheath 130 is positioned in its covering position relative to the skin-penetrating stylet 37 substantially as shown in FIG. 9.

While the sheath 130 has been depicted as an elongated body member having accordian-like pleats formed along its length, it should be understood that any other suitable means can be employed for providing a protective cover for the skin-penetrating stylet 37 when same is removed from the fluid passageway 28 of the catheter 22, such as a telescoping sheath and the like. For example, in FIG. 10 the sheath 130 is illustrated as having a compression spring 140 embedded within the body member 132 so as to enhance the movement of the body member 132 to its extended stylet covering position (FIG. 9) when the skin-penetrating stylet 37 is removed from the fluid passageway 28 of the catheter 22.

From the foregoing description, it becomes apparent that the closed system angiocatheter 10 of the present invention represents an advancement in the state of the art for numerous reasons, including, but not limited to:

(1) providing a barrier for patients from air, germs, viruses and the like during intravenous catheter set-up and use;

(2) providing a barrier to health care workers from potentially infectious blood associated with intravenous catheter use;

(3) minimizes and reduces the number of times a patient has to be stuck with a stylet or needle;

(4) minimizes potential contact with blood and/or blood products by the health care worker during intravenous setup and transfer or intravenous removal; and

(5) minimizes potential contact with an unprotected, potentially contaminated sharp puncture tip of a skin- penetrating stylet after skin-entry by health care workers upon removal of the skin-penetrating stylet from the implanted catheter.

It will be clear that the present invention is well adapted to carry out the objects and attain the advantages mentioned as well as those inherent therein. While a presently preferred embodiment of the invention has been described for purposes of this disclosure, numerous changes can be made which will readily suggest themselves to those skilled in the art and which are encompassed within the spirit of the invention disclosed and as defined in the appended claims.

## Claims

1. An improved closed system intravenous catheter positionable within a vein via a puncture site formed by a puncture tip of a-skin-penetrating stylet, the improved closed system catheter comprising:

a tapered body member having a proximal end portion and a distal end portion, the proximal end portion adapted to be inserted in the vein, the tapered body member having a fluid-flow bore extending therethrough;

a hub defining a cavity therein, the hub connected to the distal end portion of the tapered body member such that the cavity therein fluidly communicates with the fluid-flow bore of the tapered body member; and

resilient gasket means supported within the cavity of the hub for sealing the cavity of the hub and the fluid-flow bore of the tapered body member from the atmosphere.

2. The improved closed system catheter of claim 1 wherein the skin-penetrating stylet having the puncture tip formed at its proximal end is adapted to penetrate the resilient gasket means and extend through the fluid-flow bore of the tapered body member such that the puncture tip of the stylet extends from the proximal end portion of the tapered body member, the gasket means effectively sealing the fluid-flow bore of the tapered body member and the cavity in the hub when the stylet is removed therefrom.

3. The improved closed system catheter of claim 2 wherein the tapered body member and hub are of unitary construction and wherein the resilient gasket means is fabricated of a substantially fluid-impervious elastomeric material.

4. The improved closed system catheter of claim 3 wherein the tapered body member and hub are fabricated of a polymeric material.

5. An improved angiocatheter comprising:
    a catheter having a tapered proximal end portion and a hub portion, the catheter having a fluid-flow passageway extending therethrough;
    gasket means disposed within the hub portion of the catheter for sealing off the fluid-flow passageway from the outside environment; and
    a skin-penetrating stylet positionable within the fluid- flow passageway of the catheter and having a puncture tip at its proximal end for puncturing a puncture site in a patient's skin and for permitting insertion of the tapered proximal end portion of the catheter in a vein, the skin-penetrating stylet disposed within the fluid-flow passageway of the catheter so as to extend through the gasket means such that the puncture tip thereof extends outwardly from the tapered proximal end portion of the catheter, the skin-penetrating stylet slidably disposed in the fluid-flow passageway of the catheter and removable therefrom after proper placement of the catheter into the vein, the gasket means closing off the entry port made by the skin-penetrating stylet so as to seal the fluid-flow passageway of the catheter.

6. The improved angiocatheter of claim 5 wherein the skin- penetrating stylet is further characterized as having a distal end and a fluid-flow bore extending between the puncture tip and the distal end thereof, and wherein the skin-penetrating stylet further comprises:
    an observation chamber defining a flashpoint cavity therein, the observation chamber having a first end and an opposed second end, the first end of the observation chamber connected to the distal end of the skin-penetrating stylet such that a fluid-tight seal is formed therebetween and fluid communication is provided between the fluid-flow bore of the skin-penetrating stylet and the flashpoint cavity; and
    vent means supported by the opposed second end of the observation chamber for controlling blood flow into the flashpoint cavity when the skin-penetrating stylet is disposed within the catheter and the catheter is positioned in the vein.

7. The improved angiocatheter of claim 6 wherein the vent means comprises:
    a vent stylet having a first end portion and an opposed second end portion, the vent stylet connected to the observation chamber such that the first end thereof extends into the flashpoint cavity of the observation chamber and the opposed second end extends outwardly from the observation chamber;
    an air-permeable, liquid-impermeable frit supported by the opposed second end of the observation chamber so as to be disposed about an adjacently disposed portion of the vent stylet; and
    elastomeric cover means supported by the frit for enclosing the opposed second end portion of the vent stylet and forming a fluid-tight seal therebetween.

8. The improved angiocatheter of claim 7 wherein the distal end of the vent stylet is provided with a puncture tip and wherein the cover means is a substantially air-impervious elastomeric member having a closed second end disposed substantially adjacent the puncture tip of the vent stylet, the closed second end thereof penetrable by the puncture tip of the vent stylet when the elastomeric member is compressed, the elastomeric member effectively sealing the vent stylet when the elastomeric member returns to a non-compressed condition, and wherein the angiocatheter further comprises connector means supported on the opposed second end of the observation chamber for connecting auxiliary equipment to the angiocatheter.

9. The improved angiocatheter of claim 8 further comprising vent capping means disposable peripherially over the vent stylet and the elastomeric member for protecting health care workers from inadvertent contact with the vent stylet and for protecting the vent stylet from damage.

10. The improved angiocatheter of claim 5 wherein the catheter and the observation chamber of the skin-penetrating stylet are fabricated of a polymeric material.

11. The improved angiocatheter of claim 8 further comprising:

sheath means positionable over the skin-penetrating stylet for selectively covering the skin-penetrating stylet and puncture tip thereof when the skinpenetrating stylet is withdrawn from the fluid-flow passageway of the catheter.

12. The improved angiocatheter of claim 11 wherein the sheath means is connected to the observation chamber so as to extend along the skin-penetrating stylet, the sheath means selectively movable between an extended position and a retracted position, in the extended position the sheath means having a length greater than the length of the skin-penetrating stylet so that the skin-penetrating stylet and puncture tip thereof is substantially covered by the sheath means, in the retracted position the sheath means abuttingly engages the gasket member of the catheter such that the skin-penetrating stylet is positionable throughout the fluid-flow passageway of the catheter and the puncture tip of the skin-penetrating stylet extends outwardly from the tapered proximal end of the catheter.

13. The improved angiocatheter of claim 5 further comprising:

sheath means positionable over the skin-penetrating stylet for selectively covering the skin-penetrating stylet and puncture tip thereof when the skinpenetrating stylet is withdrawn from the fluid-flow passageway of the catheter.

14. The improved angiocatheter of claim 13 wherein the sheath means is connected to the observation chamber so as to extend along the skin-penetrating stylet, the sheath means selectively movable between an extended position and a retracted position, in the extended position the sheath means having a length greater than the length of the skin-penetrating stylet so that the skin-penetrating stylet and puncture tip thereof is substantially covered by the sheath means, in the retracted position the sheath means abuttingly engages the gasket member of the catheter such that the skin-penetrating stylet is positionable throughout the fluid-flow passageway of the catheter and the puncture tip of the skin-penetrating stylet extends outwardly from the proximal end of the catheter.

Fig. 1

Fig. 2

Fig. 3

Fig. 4

Fig. 5

Fig. 6

Fig. 7

Fig. 8

Fig. 9

Fig. 10

European Patent Office

**EUROPEAN SEARCH REPORT**

Application Number

EP 91 40 0879

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | US-A-4512766 (VAILANCOURT)<br>* column 2, line 41 - column 3, line 14; claims 1, 2; figures 1, 2 * | 1-6, 10 | A61M25/00<br>A61M39/00 |
| A | | 7, 8 | |
| Y | EP-A-0353905 (CRITIKON, INC.)<br>* page 1, lines 1 - 16 *<br>* page 3, lines 15 - 38 *<br>* page 4, line 28 - page 36; figure 2 * | 1-8,<br>10-14 | |
| Y,P | EP-A-0415653 (H.G.WALLACE,LTD.)<br>* column 2, line 34 - column 3, line 10 *<br>* column 4, lines 27 - 35; claims 1, 3; figure 1 * | 1-8,<br>10-14 | |
| Y | DE-A-3210964 (WOLF)<br>* pages 4 - 5, line 23; figures 1, 2 * | 5-8 | |
| Y | WO-A-8702254 (PHYSIONIC GESELLSCHAFT FUR MEDIZIN- UND SYSTEMTECHNIK GMBH)<br>* page 1, lines 5 - 10; figures 1-3 * | 10-14 | |
| | | | TECHNICAL FIELDS SEARCHED (Int. Cl.5) |
| A | US-A-3865236 (RYCROFT)<br>* abstract; figure 1 * | 5, 9 | A61M |

The present search report has been drawn up for all claims

| Place of search | Date of completion of the search | Examiner |
|---|---|---|
| BERLIN | 26 JULY 1991 | MICHELS N. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                   
& : member of the same patent family, corresponding document

EPO FORM 1503 03.82 (P0401)

13